Europäisches Patentamt

European Patent Office

Office européen des brevets

(19)

(11) Publication number: **0 317 036**
**A1**

(12) **EUROPEAN PATENT APPLICATION**

(21) Application number: 88202605.7

(22) Date of filing: 18.11.88

(51) Int. Cl.⁴: **C07H 3/02 , C12P 19/02 ,
//(C12P19/02,C12R1:645),
(C12P19/02,C12R1:01)**

(30) Priority: 20.11.87 GB 8727221

(43) Date of publication of application:
24.05.89 Bulletin 89/21

(84) Designated Contracting States:
AT BE CH DE ES FR GB GR IT LI NL SE

(71) Applicant: **UNILEVER NV**
**Burgemeester s'Jacobplein 1 P.O. Box 760**
**NL-3000 DK Rotterdam(NL)**

(84) **BE CH DE ES FR GR IT LI NL SE AT**

Applicant: **UNILEVER PLC**
**Unilever House Blackfriars P.O. Box 68**
**London EC4P 4BQ(GB)**

(84) **GB**

(72) Inventor: **Cheetham, Peter Samuel James**
**59 Prospect Way Brabourne Lees**
**Ashford Kent TN25 6RL(GB)**
Inventor: **Meakins, Stephen Edwin**
**12 Longacre Close**
**Canterbury Kent CT2 7TE(GB)**

(74) Representative: **Wiesenhaan, Herman, Drs. et al**
**Unilever N.V. Patent Division P.O.Box 137**
**NL-3130 AC Vlaardingen(NL)**

(54) **Glycoside hydrolysis.**

(57) The invention provides a process for preparing L-rhamnose by hydrolysing glycosidic bonds in a glycoside having rhamnose in a terminal position by heating in an aqueous solution of an organic carboxylic acid at a pH between 1 and 3. Preferably the organic carboxylic acid is acetic acid, propionic acid, citric acid or malic acid in the form of a solution of the organic acid contains 0.1-25% (w/w) of organic acid. Also the glycoside can be used in the form of crude citrus material.

EP 0 317 036 A1

## GLYCOSIDE HYDROLYSIS

This invention relates to a process for hydrolysing a glycoside having rhamnose in a terminal position so as to obtain free rhamnose. Rhamnose is a valuable intermediate for the preparation of, inter alia, pharmaceutical and flavour compounds, but rhamnose previously has been accessible only with difficulty and by means of routes which cannot be considered to involve only natural ingredients and natural processes. Moreover, the known processes also employ scarce and/or expensive enzyme materials.

Processes to obtain free rhamnose from glycoside material are known from the prior art. Japanese patent application JP-A-62/292 (Kanegafuchi Chem.Ind.Co.) discloses a process for hydrolysing flavonoid glycoside material by means of prolonged heating in an aqueous solution of a strong mineral acid, removal of residual flavonoid compounds, followed by digestion of accompanying glucose with enzymes or bacteria and recovery of L-rhamnose from the resulting solution. Also there is Japanese patent application JP-A 62/293 (Kanegafuchi Chem. Ind. Co.) which discloses a process for selectively cleaving the rhamnoside bond of a rhamnose-containing flavonoid compound by the action of a suitable enzyme in an aqueous solution at a low pH, adjusted by adding mineral acid, after which the residual flavonoid compound is removed by precipitation and L-rhamnose is recovered from the resulting solution. Both methods involve mineral acid, extreme conditions and optionally employ specific enzymes. Elaborate purification steps are consequently necessary to remove mineral acid, enzyme and undesirable degradation products.

Also there is J. Agric. Food Chem. 29, N° 6, (1981), 1299, describing the heating to near boiling of 3.2 g naringin in 4.0 l of tap water containing 190 g of sucrose and 1 g of citric acid, but this merely produced a solution of naringin in water.

The present invention provides a natural process for the preparation and recovery of rhamnose employing mild, natural reagents and conditions that yield pure L-rhamnose by carrying out the hydrolysis of a glycoside having rhamnose in a terminal position in an aqueous solution of an organic acid, preferably a carboxylic acid. This hydrolysis is preferably followed by removal of any glucose liberated, thereby obtaining an aqueous solution of rhamnose from which rhamnose can by crystallised and collected. In order to minimize the amounts of glucose that must removed, conditions of hydrolysis are chosen that are selective and allow high yields of rhamnose to be obtained, while minimising the amounts of glucose released.

Hydrolysis of glycoside starting materials, such as quercitin, naringin, hesperidin, neohesperidin and rutin, or crude natural material containing these compounds, like citrus waste materials and crude extracts of rue, fenugreek, gum acacias (such as kordofan, talha etc), microbial rhamnolipids, and microbial rhamnopoly-saccharides e.g. from Xanthomonas Arcc 53159 can be conveniently effected in an aqueous 0.1-25% solution of a carboxylic acid containing 1 to 6 carbon atoms. Suitable organic acids are food grade organic acids like citric acid, lactic acid, malic acid, succinic acid, acetic acid, propionic acid, tartaric acid, fumaric acid, etc. Preferably, because of their activity and selectivity acetic acid and/or propionic acids are used, for easier processing citric and/or malic acid are also preferred.

For efficient hydrolysis, it is desirable that the heating takes place for a period between 1 and 10 hours at a temperature between 80 and 120°C. Reaction temperatures above 100°C can be conveniently obtained in a pressure cooker at superatmospheric pressures. Under these conditions, virtually complete liberation of the rhamnose is obtained, but still it may be desirable to remove some of the unconverted starting material and flavonol compounds, e.g. by filtering after cooling.

The hydrolysate so obtained contains rhamnose usually together with some glucose and some flavones and flavone glucosides and organic acid, and is preferably further purified so as to remove glucose, the flavone and flavone glucosides and optionally organic acid. Convenient methods for this are:

1. Selective fermenting out of glucose with a suitable microorganism under conditions chosen to preferentially ferment glucose, rather than rhamnose ethanol being produced as a side product. Such microorganisms are e.g. Saccharomyces cerevisiae, Candida albicans, Lactobacillus delbruckii and Proteus bulgaris. Preferably the fermentation is carried out at pH 4 - 4.5. No selective fermentation was observed when the pH of the syrup was higher e.g. pH 6.0. Preferably, the microorganism, in particular a yeast, has been immobilised e.g. in calcium alginate, which can optionally be dried prior to use to improve their mechanical and storage properties, and the hydrolysate is pumped through a column packed with immobilised microorganism. In addition free cells or immobilized cells can be in a stirred batch reaction. The activities are ca 1.9 and 3.4 gram of glucose- free rhamnose per litre of reactor content per hour respectively; and for immobilized cells used in a column 22.4 gram glucose-free- rhamnose/litre/hour. Some of the microorganisms are also able to ferment out the organic acid so that a virtually pure rhamnose solution is obtained. Also, the organic acid can be precipitated as an inorganic salt (especially the calcium salt) and removed. Most of the rhamnose lost was metabolized during the start of the fermentation. Varying the sugar concentration in the syrup has no effect on the yield of rhamnose, which is about 65 % when all the glucose has been removed, but the presence of lower glucose concentration results in a proportionally lower fermentation time, therefore a proportionally higher flow rate can be used. Higher yields of rhamnose are obtained if some residual glucose can be tolerated e.g. 74 %, 82 % and 92.5 % recoveries of rhamnose respectively at 1 %, 2 % and 6 % (w/w) residual glucose. The immobilized cells can be reused, but their activities may decrease with time.

2. Alternatively glucose can be removed by the selective enzymic oxidation of glucose to gluconic acid (using glucose oxidase and catalase), which is subsequently precipitated as its calcium salt, optionally together with the food grade organic acid, and removed. The glucose oxidase/catalase may also be used in an immobilized form. Sources of the enzyme that are most resistant to inhibition by product are preferred. The time at which the calcium source is added is not critical, indeed it can be present with the glucose/catalase from the start to precipitate gluconic acid as it is formed, although this option is not preferred. Glucose dehydrogenase may also be used instead of glucose oxidase. Recoveries of rhamnose up to 100% can be obtained by this method whilst completely removing the glucose. Gluconic acid is produced as a side-product from the glucose.

3. Selective oxidation of glucose to 5-ketogluconic acid by means of a suitable microorganism and separate recovery of 5-ketogluconic and rhamnose. One preferred microorganism for carrying out this oxidation is <u>Gluconobacter oxydans</u>. This method, however, does not work if the glucose concentration in solution is above 6-7 % (w/v) since glucose is inhibitory at high concentrations. Therefore preferably only dilute sugar solutions are treated by the method. Recoveries of rhamnose free of glucose, up to 95% have been obtained by the method. Also the calcium salt of 5-ketogluconic acid is produced as a side-product.

4. Selective adsorption/absorption of either the glucose or the rhamnose e.g. using activated charcoal or bone-charcoal to preferentially absorb the rhamnose. This method is best carried out by using the carbon in a chromatographic mode.

For processing reasons, it has been found convenient to select an organic acid in the hydrolysis step that does not interfere with the further processing, e.g. one selects an organic acid that is fermented out during glucose fermentation. This simplifies further purification and recovery.

Further optional purification steps comprise cooling the hydrolysate to precipitate impurities followed by the absorption, precipitation of impurities and crystallization of L-rhamnose. The most preferred absorbants and precipitants were activated charcoal, an ion exchange resin such as Amberlite XAD-4 (Amberlite is a tradename) and polyvinylpolypyrrolidone. Dosage-purification relationships were studied and it was established that optimal use levels were 3%, 17.5% and 5% respectively. Carbon treatment was optimally carried out in hot syrup of ca 80.C.

The natural rhamnose so obtained is of high purity and very suitable for use for further conversions to pharmaceutical and flavour compounds.

In particular, this grade of rhamnose can be converted into flavour compounds such as 2,5-dimethyl-4-hydroxy-2,3-dihydrofuran-3-one.

The invention is illustrated by the following examples:

## Example 1

A 10 % (w/w) slurry of naringin in a 10% (w/w) aqueous solution of acetic acid at pH 2.2, was stirred to 120°C for 5 hours. After cooling and filtration, a solution was obtained which contained 100% of the theoretical quantity of rhamnose present in naringin. (28.3 g rhamnose /100 g naringin) and 25 % of the theoretical quantity of glucose. Analysis was done by hplc versus a 1 % (w/w) rhamnose monohydrate : 1 % (w/w) glucose monohydrate standard.

The pH of this solution was then raised to 4 by addition of $Ca(OH)_2$, to allow the efficient action of the glucose oxidase. Care was taken not to allow the pH to rise above pH 6.0 in which case the naringin becomes soluble and cannot be easily removed by filtration. The resulting filtrate was treated with 10 % (w/v) of polyvinylpolypyrrolidone (p.v.p.) and 5 % (w/v) charcoal to remove any remaining impurities. Glucose was removed using glucose oxidase in combination with catalase. From the resulting solution rhamnose crystals could be obtained by concentrating and cooling. The results are tabulated below:

| STEP | WEIGHT OF LIQUOR in g | %RHA | %GLU | WEIGHT RHAMNOSE in g |
|------|------------------------|------|------|----------------------|
| START (NARINGIN) powder | 120 | 28.3 | 31.0 | 33.96 |
| | | | | (present as glycoside) |
| HYDROLYSIS | 800 | 4.2 | 1.16 | 33.60 |
| AFTER Ca(OH)$_2$ TREATMENT | 1170 | 2.89 | 0.77 | 32.90 |
| AFTER PVP TREATMENT | 843 | 3.47 | 0.94 | 29.28 |
| AFTER CARBON TREATMENT | 817 | 2.77 | 0.94 | 22.69 |
| AFTER ENZYME TREATMENT & CRYSTALLISATION | 812 | 2.83 | 0.00 | 23.00 |
| | | | | (68% overall yield) |

Example 2

A 15% (w/w) slurry of naringin in a 10% (w/w) aqueous solution of citric acid was heated at 120°C for three hours. After cooling just sufficient calcium hydroxide was added to neutralise the citric acid used. This mixture was then further cooled to 4°C before being filtered. The filtrate obtained contained 80% of the theoretical quantity of rhamnose present in naringin, and 55% of the theoretical quantity of glucose. This filtrate was then treated with charcoal (1.5% w/w) at 80°C for two hours, before being re-filtered. The pH of this solution was then adjusted to 5.5 by the careful addition of calcium hydroxide, and it was incubated with glucose oxidase (0.5 w/w) in combination with catalase (0.5% w/w). When all of the glucose had been consumed calcium carbonate (3.0% w/w) was added. The pH of the solution was then raised to 7.0 by the addition of calcium hydroxide and the precipitated calcium gluconate removed by filtration.

The filtrate obtained was then heated at 80°C for two hours with charcoal (1.5% w/w) cooled and filtered. This solution was then concentrated to one fifth of its original volume and added to 96% ethanol (twice the volume of concentrate obtained) which precipitated any organic salts or protein left in solution. After filtration the ethanol was evaporated to give rhamnose as a white amorphous powder.

The results are tabulated below:

| STEP | WEIGHT OF LIQUOR in g | %RHA | %GLU | WEIGHT RHAMNOSE in g |
|------|------------------------|------|------|----------------------|
| START (NARINGIN) powder | 120 | 28.3 | 31.0 | 33.96 |
| | | | | (present as glycoside) |
| HYDROLYSIS | 800 | 3.37 | 2.56 | 27.00 |
| AFTER Ca(OH)$_2$ TREATMENT | 730 | 3.70 | 2.80 | 27.00 |
| AFTER CARBON TREATMENT | 715 | 3.70 | 2.80 | 26.45 |
| AFTER ENZYME TREATMENT | 700 | 3.40 | 0.00 | 23.80 |
| AFTER CARBON TREATMENT | 658 | 3.10 | 0.00 | 20.34 |
| AFTER ETHANOL TREATMENT & CRYSTALLISATION | 24.1 | 76.3 | 0.00 | 18.38 |
| | | | | (54% overall yield) |

Example 3

Using malic acid as in Example 2 yields of rhamnose and glucose obtained following hydrolysis were 3.38 and 3.10% (w/w) respectively and following purification pure rhamnose was obtained in a 69% yield.

Examples 4-40

| Ex.No. | Rhamnose source % | | Acid used % | | Hydrolysis % theor. yield | | |
|---|---|---|---|---|---|---|---|
| | | | | | Rha | Glu | Rha/G |
| 4 | Naringin | (10) | Acetic | (10) | 100 | 38 | 2.5 |
| 5 | Naringin | (15) | Acetic | (10) | | 33 | 2.0 |
| 6 | Naringin | (20) | Acetic | (10) | 80 | 26 | 3.1 |
| 7 | Naringin | (40) | Acetic | (10) | 67 | 15 | 4.5 |
| 8 | Naringin | (40) | Acetic | (20) | 84 | 24 | 3.5 |
| 9 | Naringin | (10) | Citric | (10) | 100 | 92 | 1.1 |
| 10 | Naringin | (10) | Citric | (10) | 96 | 73 | 1.35 |
| 11 | Naringin | (10) | Malic | (10) | 100 | 67 | 1.5 |
| 12 | Naringin | (10) | Malic | (20) | 96 | 50 | 1.7 |
| 13 | Naringin | (10) | Tartaric | (10) | 100 | 86 | 1.2 |
| 14 | Naringin | (10) | Lactic | (10) | 100 | 100 | 1.0 |
| 15 | Naringin | (10) | Propionic | (10) | 80 | 22 | 3.6 |
| 16 | Naringin | (10) | Butyric | (10) | 66 | 11 | 6.0 |
| 17 | Naringin | (10) | Caproic | (10) | 34 | 3.5 | 9.7 |
| 18 | Naringin | (10) | Caprylic | (10) | 11 | 0 | - |
| 19 | Naringin | (10) | Glutamic | (10) | 59 | 13 | 4.5 |
| 20 | Naringin | (10) | Formic | (10) | 84 | 55 | 1.5 |
| 21 | Naringin | (10) | Succinic | (10) | 75 | 27 | 2.8 |
| 22 | Rutin | (10) | Citric | (10) | 95 | 100 | 0.95 |
| 23 | Rutin | (10) | Citric | ( 5) | 68 | 70 | 1.0 |
| 24 | Rutin | (10) | Citric | ( 1) | 44 | 45 | 1.0 |
| 25 | Rutin | (10) | Tartaric | (10) | 37 | 40 | 0.9 |
| 26 | Rutin | (10) | Lactic | (10) | 71 | | - |
| 27 | Rutin | (10) | Acetic | (10) | 37 | 47 | 0.8 |
| 28 | Rutin | (10) | Propionic | (10) | 23 | 30 | 0. |
| 29 | Quercitrin | (10) | Acetic | (10) | 91 | 0 | - |
| 30 | Citrus wastes (10) | | Citric | (10) | 75 | 80 | 0. |
| 31 | Citrus wastes (10) | | Mali | (10) | 82 | 85 | 1. |
| 32 | Citrus wastes (10) | | Tartaric | (10) | 89 | 90 | 1. |
| 33 | Citrus wastes (10) | | Lactic | (10) | 60 | 60 | 1. |
| 34 | Citrus wastes (10) | | Acetic | (10) | 31 | 35 | 0. |
| 35 | Citrus peel sweet (10) | | Citric | (10) | 54 | na | - |
| 36 | Citrus peel bitter (10) | | Lactic | (10) | 54 | na | - |
| 37 | Gum kordof. (30) | | Acetic | (10) | 82 | 0 | - |
| 38 | Gum kordof. (30) | | Propionic | (10) | 59 | 0 | - |
| 39 | Gum kordof. (30) | | Citric | (10) | 100 | 0 | - |
| 40 | Gum talha (30) | | Citric | (10) | 100 | 0 | - |

Example 41

Selective fermentation of glucose present in purified rutin hydrolysate obtained according to Example 17 was carried out by using bakers' yeast (Saccharomyces cerevisiae) immobilized in calcium alginate pellets which were used in columns with the syrup pumped through continuously.

60 g of bakers' yeast paste was mixed with 40 ml of 3 % (dry weight per volume) sodium alginate solution and extruded dropwise into 0.1 M aqueous calcium chloride solution. After 30 minutes incubation at room temperature, the pellets were packed into a 100 ml column and substrate, adjusted to pH 4.5 with citric acid was pumped through the column at a flow rate of ca 0.05 column volume/hour.

The eluate was analyzed for sugars by hplc. The operational lifetime of the column was about two weeks. The column was run so that only 1-2 % residual glucose remained, which was subsequently easily removed by crystallization of the rhamnose. The glucose fermented was converted into ethanol in a 40% molar yield. Rhamnose syrup, essentially free of glucose and containing about 20 % w/v of rhamnose was treated with activated charcoal (20 g/1) to remove impurities. The syrup (1 litre) was concentrated by rotary evaporation at 60-70° C. to about 65 % (w/v) and then allowed to cool. Crystals were isolated by filtration and then dried at 40° C. The yield of crystals obtained was proportional to the concentration, which was up to about 70 % (w/w), at which concentration the viscosity of the syrup became excessive and crystals became contaminated with glucose. The mother liquor syrup was again concentrated by rotary evaporation to about 70 % (w/w), allowed to cool and a second crop of crystals filtered and subsequently recrystallized. The yield of pure glucose-free L-rhamnose monohydrate crystals was 86 % based on the amount of rhamnose present in the original syrup. Crystalline rhamnose produced by this method was stored for over 4 months at 20° C and showed no signs of deterioration.

## Example 42

A sample of the glucose/rhamnose syrup produced according to Example 24 using citrus waste material yielding 200 ml syrup at 5 % of sugars was purified using p.v.p., then charcoal and adjusted to pH 6.5 and 0.5 ml each of glucose oxidase and catalase added. The syrup was then incubated at 35° C until all the glucose had disappeared, as measured by hplc analysis, which was after 72 hours. Calcium hydroxide (20 ml, 5% w/v solution) was then added to precipitate the gluconic acid as calcium gluconate. The resulting pure rhamnose solution was decanted and filtered through a diatomaceous earth filter aid to remove residual traces of calcium gluconate and the rhamnose was recovered in good yield, 180 ml of 2.62 % w/v solution of rhamnose i.e. 78.7 % theoretical yield.

EP 0 317 036 A1

**REMOVAL OF GLUCOSE FROM RHAMNOSE/GLUCOSE SYRUPS BY OXIDATION USING THREE DIFFERENT GLUCOSE OXIDASES**

**Examples 43-48 plus illustrative experiments**

| TYPE OF GLUCOSE OXIDASE | | BIOCON | | SIGMA II-S | | BIOCATALYSIS OF | |
|---|---|---|---|---|---|---|---|
| Starting syrup | Enzymes added to syrup | Time to zero glucose | Rhamnose yield | Time to zero glucose | Rhamnose yield | Time to zero glucose | Rhamnose yield |
| 200 g/l glucose 200 g/l rhamnose | 4% (v/v) glucose oxidase 2% (v/v) catalase | 48 hrs | 92% | down to 20 g/l glucose in 2 hrs | 97.5% | 30 hrs | 100% |
| 20 g/l glucose 50 g/l rhamnose | 0.4% (v/v) glucose oxidase 0.2% (v/v) catalase | < 5 hrs | 90% | < 5 hrs | 100% | < 5 hrs | 100% |
| 200 g/l glucose | 4% (v/v) glucose oxidase | < 5 hrs | N/A | down to 25 g/l glucose in 5 hrs | N/A | < 5 hrs | N/A |
| 20 g/l glucose | 0.4% (v/v) glucose oxidase 0.2% (v/v) catalase | < 5 hrs | N/A | < 5 hrs | N/A | > 5 hrs | N/A |
| 50 g/l rhamnose | 1.0% (v/v) glucose oxidase 0.5% (v/v) catalase | N/A | 90% after 120 hrs | N/A | 90% after 120 hrs | N/A | 90% after 120 hrs |

Examples 43 - 48

The procedure of the previous example was repeated with different starting materials and enzymes. The results are tabulated below. The speed of the reaction was mainly dependent on the concentration of flavones and flavanoids remaining.

| Starting syrup | Enzymes added | Incubation time | Rha yield |
|---|---|---|---|
| 200 g/l glucose<br>200 g/l rhamnose | 4%(v/v) gluc oxidase (Biocon)<br>2%(v/v) catalase (BDH) | 48 hours | 92% |
| 20 g/l glucose<br>50 g/l rhamnose | 0.4%(v/v) gluc oxidase (Biocon)<br>0.2%(v/v) catalase (BDH) | < 5 hours | 90% |
| 200 g/l glucose<br>200 g/l rhamnose | 4%(v/v) gluc oxidase (Sigma II)<br>2%(v/v) catalase (BDH) | at 20 g/l<br>gluc 2 hrs | 97% |
| 20 g/l glucose<br>50 g/l rhamnose | 0.4%(v/v)gluc oxidase (SigmaII)<br>0.2% (v/v) catalase (BDH) | < 5 hours | 100% |
| 200 g/l glucose<br>200 g/l rhamnose | 4%(v/v) gluc oxidase (Biocat)<br>2%(v/v) catalase (BDH) | 30 hours | 100% |
| 20 g/l glucose<br>50 g/l rhamnose | 0.4%(v/v) gluc oxidase (Biocat)<br>0.2%(v/v) catalase (BDH) | < 5 hours | 100% |

BDH = ex British Drug Houses, Biocat = Biocatalysts type SF

Example 49

From various examples given previously a mixture of rhamnose and glucose was obtained. According to the present example the glucose was converted into 5-ketogluconic acid, which could be easily separated from the rhamnose. The conversion was effected by the organism Gluconobacter oxydans . The 5-ketogluconic acid formed was precipitated as its calcium salt and subsequently recovered. 10 ml of a glucose/rhamnose syrup containing 220 g/l of glucose and 220 g/l of rhamnose were diluted with 87 ml of water, 3 % of calcium carbonate were added as buffer and as to provide calcium ions. The pH was adjusted to 6.5. 1.2 g of previously grown Gluconobacter oxydans wet cells were added and incubation took place at 30° C for 72 hours whilst shaking. A precipitate of the calcium salt of 5-ketogluconic acid was formed and removed by filtration together with the bacteria. Further separation yielded 2.0 g of rhamnose and 2.0 g of 5-ketogluconic acid. If fermentation was continued after the point at which all the glucose had been converted, then the rhamnose was then metabolised.

Example 50

Glucose and rhamnose were separated by adsorption of the rhamnose onto activated carbon and washing the rhamnose off the carbon. A solution prepared according to Example 1 and concentrated, containing 10 % of rhamnose and 12 % of glucose per litre was treated with 10 % w/w of powdered activated carbon. 11.8 % of the rhamnose was adsorbed on the carbon, but only 3 % of the glucose. More effective separation was achieved by repeating this process.

## Claims

1. A process for preparing L-rhamnose by hydrolysing glycosidic bonds in a glycoside having rhamnose in a terminal position, characterised in that the glycoside is hydrolysed by heating in an aqueous solution of an organic carboxylic acid at a pH between 1 and 6, preferably between 1 and 3.

2. A process according to claim 1, characterised in that the organic carboxylic acid contains 1 - 6 carbon atoms.

3. A process according to claim 1 or 2, characterised in that the solution of the carboxylic acid contains 0.1 - 25% (w/w) of carboxylic acid:

4. A process according to any of the preceding claims characterized in that the glycoside is selected from the group consisting of rutin, naringin, quercitrin, gum acacia, microbial rhamnolipid and microbial rhamnopolysaccharide.

5. A process according to any of the preceding claims characterized in that the glycoside is in the form of crude citrus material.

6. A process according to any of the preceding claims characterised in that hydrolysis takes place to a temperature between 80 and 120° C.

7. A process according to any of the preceding claims, characterised in that hydrolysis lasts for 1-10 hours.

8. A process according to any of the preceding claims characterised in that the reaction mixture is heated at superatmospheric pressure.

9. A process according to any of the preceding claims, characterised in that the hydrolysis is followed by a purification step of the hydrolysate.

10. A process according to claim 9, characterised in that any glucose present is fermented out of the hydrolysate with a microorganism, preferably with an immobilised yeast, at pH 3 - 5.

11. A process according to claim 9, characterised in that any glucose present is oxidised to gluconic acid or 5-ketogluconic acid which is then removed.

12 A process according to claim 11, in which the conversion is carried out using Gluconobacter oxydans.

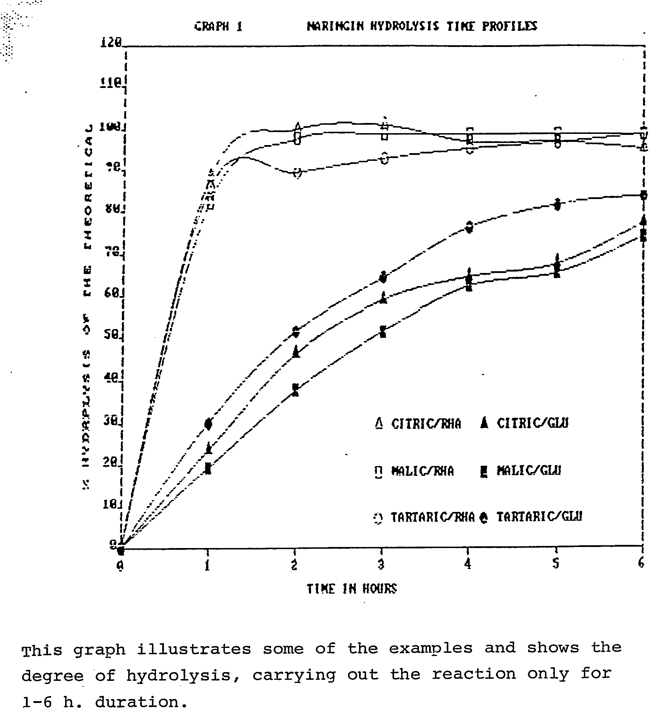

GRAPH 1     NARINGIN HYDROLYSIS TIME PROFILES

Δ CITRIC/RHA    ▲ CITRIC/GLU

□ MALIC/RHA    ▇ MALIC/GLU

◌ TARTARIC/RHA    ● TARTARIC/GLU

TIME IN HOURS

This graph illustrates some of the examples and shows the degree of hydrolysis, carrying out the reaction only for 1-6 h. duration.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,Y | US-A-3 429 873 (R.M. HOROWITZ)<br>* Whole document *<br>--- | 1-12 | C 07 H    3/02<br>C 12 P   19/02 //<br>(C 12 P   19/02 |
| Y | THE JOURNAL OF THE AMERICAN CHEMICAL SOCIETY, vol. 61, January-June 1939, pages 175-176; G.N. PULLEY et al.: "Preparation of rhamnose from naringin"<br>* Whole article *<br>--- | 1-12 | C 12 R    1:645)<br>(C 12 P   19/02<br>C 12 R    1:01 ) |
| A | CHEMICAL ABSTRACTS, vol. 34, no. 11, 10th June 1940, column 3694 (5-9), Columbus, Ohio, US; S. MURAKAMI: "Constitution of verbascose, a new pentasaccharide", & PROC. IMP. ACAD. (TOKYO) 16, 12-14(1940)<br>* Abstract *<br>--- | 1 | |
| A,D | J. AGRIC. FOOD CHEM., vol. 29, 1981, pages 1298-1301, American Chemical Society; G.M. GRAY et al.: "Hydrolysis of high levels of naringin in grapefruit juice using a hollow fiber naringinase reactor"<br>* Whole article *<br>----- | 1 | TECHNICAL FIELDS SEARCHED (Int. Cl.4)<br><br>C 07 H   3/00<br>C 07 H  17/00 |

The present search report has been drawn up for all claims

| Place of search | Date of completion of the search | Examiner |
|---|---|---|
| THE HAGUE | 23-02-1989 | BRENNAN J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)